# EUROPEAN PATENT APPLICATION

(11) **EP 1 862 081 A1**
(43) Date of publication of application: **05.12.2007**
(21) Application number: 06715598.6
(22) Date of filing: 13.03.2006
(51) Int. Cl.: A23K 1/175, A01K 67/00, A23K 1/18

(54) **FEED COMPOSITION AND METHOD OF BREEDING ANIMAL**

(30) Priority: 25.03.2005 JP 2005089310
(71) Applicant: KENKO CORPORATION, Tokyo, 1010047 (JP); Sinanen Zeomic Co., Ltd., Minato-ku Nagoya-shi, Aichi (JP)
(72) Inventor: TSUCHIBE, Satomi, Tokyo (JP); TAKADA, Toshiyasu, Chiba (JP); UCHIDA, Masashi, hi, Aichi, 4850812 (JP); KURIHARA, Yasuo, hi, 4670014 (JP)
(74) Representative: Hubert, Philippe
(86) International application number: PCT/JP2006/304875
(87) International publication number: WO 2006/103905

(57) **Abstract**

Objects of the invention are to provide a feed composition, which shows a high antimicrobial activity to a variety of harmful microorganisms, in particular, harmful bacteria such as those belonging to the genus Salmonella, which can show, on the contrary, only a low antibacterial effect on lactic acid bacteria useful for the growth of animals while keeping their good health and which can thus permit the breeding of animals with health, while ensuring a high breeding efficiency as well as a method for breeding animals using the feed composition. The invention provides a feed composition **characterized in that** it comprises silver-carrying zeolite; and a method for breeding an animal wherein if the feed composition having a silver content of 2.5% by mass is administered to the animal, the composition having a silver content of 0.001 to 0.2% by mass is used in the initial breeding stage (term A), the composition having a silver content of less than 0.001% by mass is used in the internal stage (term B), and the composition having a silver content of 0.01 to 0.2% by mass is used after the internal stage and before the shipping date (term C).

## Description

### Field of the invention

The present invention relates to a feed composition for use in, for instance, poultry (domestic fowls) and livestock (domestic animals) such as chickens, cattle and swine and a method for breeding an animal using the feed composition, and more specifically to a feed composition which has almost no influence upon the useful lactic acid bacteria, but exerts a strong antimicrobial effect on various kinds of harmful microorganisms, in particular, bacteria belonging to the genus Salmonella or the like, as well as a method for breeding an animal using such a feed composition.

### Background art

The consuming public has recently been concerned about the safety of livestock and aquatic products or foods such as edible meat, eggs, and fishes and shellfishes. In this respect, there have been known a variety of harmful microorganisms such as bacteria and viruses which can invade the bodies of cattle, poultry and cultivated fishes and settle in the bodies of these animals. As a result, individuals may be infected with such various kinds of microorganisms through, for instance, the intake of a food contaminated with the same, in particular, bacteria belonging to the genus Salmonella and this in turn results in the occurrence of various hygienic injuries such as food poisoning and a variety of serious diseases and this accordingly becomes an object of public concern.
There has not yet been completely elucidated a route through which various harmful microorganisms such as bacteria and viruses, in particular, bacteria belonging to the genus Salmonella causing these food poisoning and serious diseases enter into human and animal bodies. In this respect, it has in general been recognized that, in most cases, these microorganisms may enter into the bodies through various feeds or foods and/or drinking water, as mediators, ingested by these animals. In this connection, as methods for protecting animals from being poisoned by foods or from being infected with the foregoing causal bacteria or viruses for the serious diseases described above, there may be listed, for instance, a method for sterilizing feeds or foods and drinking water; or a method for imparting, to these animals, an antimicrobial activity which permits the control of any proliferation of such harmful microorganisms, for instance, pathogenic bacteria.
There has conventionally been proposed a feed composition to which an antimicrobial activity is imparted by the incorporation, into the feed composition, of a variety of antimicrobial agents such as antibiotics, sulfa drugs and iodine-containing compounds (see, for instance, Patent Document No. 1 specified below). However, these antimicrobial agents may suffer from various problems such that they show unstable antimicrobial effects on highly resistive bacteria such as those belonging to the genus Salmonella and if they enter into human bodies through the intake of a food derived from an animal bred using the antibacterial agent-containing feed composition, they may induce allergic reactions in the human bodies and/or may exert an adverse effect on the valuable intestinal bacteria such as lactic acid bacteria.

Patent Document No. 1: JP-A-2003-40717.

### Disclosure of the invention

### Problems to be solved by the invention

Accordingly, it is in general an object of the present invention to provide a feed composition containing an antimicrobial agent and a method for breeding an animal while making use of the feed composition.
More specifically, it is an object of the present invention to provide a feed composition, which shows a high antimicrobial activity to a variety of harmful microorganisms, in particular, harmful bacteria such as those belonging to the genus Salmonella, which can show, on the contrary, only a low antibacterial effect on lactic acid bacteria useful for the growth of animals while keeping their good health and which can thus permit the breeding of animals with health, while ensuring a high breeding efficiency as well as a method for breeding animals using the feed composition.

### Means for solving the problems

The present invention provides a feed composition and a method for breeding animals using the same as will be detailed below:
1. A feed composition characterized in that it comprises silver-carrying zeolite.
2. The feed composition of the above item 1, wherein the amount of silver present in the silver-carrying zeolite ranges from 0.1 to 5.0% by mass on the basis of the total mass of the silver-carrying zeolite.
3. The feed composition of the above item 1, wherein the content of silver in the composition ranges from 0.000001 to 0.2% by mass (0.01 to 2000 ppm) on the basis of the total mass of the feed composition.
4. The feed composition of the above item 1, wherein the amount of silver present in the silver-carrying zeolite ranges from 0.1 to 5.0% by mass on the basis of the total mass of the silver-carrying zeolite and the content of silver in the feed composition ranges from 0.000001 to 0.2% by mass (0.01 to 2000 ppm) on the basis of the total mass of the composition.
5. A method for breeding an animal comprising the step of administering, to an animal, a feed composition comprising silver-carrying zeolite.
6. The animal-breeding method of the above item 5, wherein the amount of silver present in the silver-carrying zeolite ranges from 0.1 to 5.0% by mass on the basis of the total mass of the silver-carrying zeolite.
7. The animal-breeding method of the above item 5, wherein the content of silver in the composition ranges from 0.000001 to 0.2% by mass (0.01 to 2000 ppm) on the basis of the total mass of the feed composition.
8. The animal-breeding method of the above item 5, wherein the amount of silver present in the silver-carrying zeolite ranges from 0.1 to 5.0% by mass on the basis of the total mass of the silver-carrying zeolite and the content of silver in the feed composition ranges from 0.000001 to 0.2% by mass (0.01 to 2000 ppm) on the basis of the total mass of the composition.
9. The animal-breeding method as set forth in any one of the above items 5 to 8, wherein the feed composition having a silver content of A% by mass (A being not less than 0.000001) is administered to the animal in the initial breeding stage.
10. The animal-breeding method as set forth in any one of the above items 5 to 8, wherein the composition having a silver content of A% by mass (A being not less than 0.000001) is administered to the animal in the initial breeding stage and the composition having a silver content of B% by mass (B<A) is administered to the animal after the completion of the initial breeding stage.
11. The animal-breeding method of the above item 10, wherein the animal is a fowl bred for egg collection or dairy cattle.
12. The animal-breeding method as set forth in any one of the above items 5 to 8, wherein the composition having a silver content of A% by mass (A being not less than 0.000001) is administered to the animal in the initial breeding stage; the composition having a silver content of B% by mass (B<A) is administered to the animal during the term extending from the end of the initial breeding stage to 5 to 20 days before the distribution or shipping date; and the composition having a silver content of C% by mass (C>B) is administered to the animal during the term of 5 to 20 days before the shipping date.
13. The animal-breeding method of the above item 12, wherein the animal is one for obtaining meat.
14. The animal-breeding method of the above item 13, wherein the animal is poultry, cattle, swine, a wild boar, an equine, sheep or a deer.
15. The animal-breeding method of the above item 13, wherein the animal is a domestic fowl for chicken, beef cattle or swine.

### Effects of the invention

The feed composition of the present invention has almost no influence upon the useful lactic acid bacteria, but can exert a substantial or strong antimicrobial effect on various kinds of harmful microorganisms, in particular, bacteria belonging to the genus Salmonella or the like. Accordingly, the feed composition would permit the improvement of the healthiness of the intestinal tracts of domestic animals, domestic fowls and cultivated fishes such as chickens, swine, cattle, equines, carps and various kinds of cultivated fishes and the composition would, in its turn, permit the acceleration of their growth. Moreover, the meat, milk, eggs and other livestock products derived from the animals bred by administering the feed composition of the present invention are completely free of any contamination with a variety of harmful microorganisms, in particular, bacteria belonging to, for instance, the genus Salmonella and therefore, they are quite safe from the viewpoint of the food sanitation.
Further, in the method of the present invention, the composition having a silver content of A% by mass (A being not less than 0.000001) is administered to the animal in the initial breeding stage or during the term extending from its birth to its childhood until the animal acquires the immunity almost comparable to that of the imago thereof and therefore, the method of the invention would permit the prevention of any infection of the animal in its childhood with various harmful microorganisms.

Furthermore, in the method of the present invention, the composition having a silver content of A% by mass (A being not less than 0.000001) is administered to the animal in the initial breeding stage and the composition having a silver content of B% by mass (B<A) is administered thereto after the completion of the initial breeding stage. Accordingly, the method of the invention permits the efficient use of expensive silver and the effective breeding of animals while maintaining the healthy conditions thereof and ensuring a high breeding efficiency. In particular, when breeding fowls for egg collection with the feed composition of the present invention, the fowls would provide eggs over a quite long period of time.
In addition, in a preferred embodiment of the method according to the present invention, the composition having a silver content of A% by mass (A being not less than 0.000001) is administered to the animal in the initial breeding stage; the composition having a silver content of B% by mass (B<A) is administered to the animal during the term extending from the end of the initial breeding stage to about one to two weeks before the distribution or shipping date of the animal or the by-products or fruits derived from the same; and the composition having a silver content of C% by mass (C>B) is then administered to the animal during the term of about one to two weeks before the shipping date thereof. Accordingly, the method of the invention permits the efficient use of expensive silver and the effective breeding of animals, in particular, those bred for obtaining meat, while maintaining the healthy conditions of the animals and ensuring a high breeding efficiency.
If using the feed composition of the present invention, it would be expected that animals can be protected from the infection with, for instance, pathogenic bacteria without using any antibiotic agent at all and this may in turn inhibit the generation of any resistant bacteria possibly generated due to frequent use of antibiotics and this likewise permits the complete protection of human bodies from the infection with such pathogenic bacteria or the reduction of the probability that individuals are infected with the bacteria.
Further, the feed composition of the present invention has almost no bad influence upon bacteria useful for animals such as lactic acid bacteria, but exerts a high antimicrobial effect on various kinds of harmful microorganisms, in particular, bacteria belonging to, for instance, the genus Salmonella and accordingly, the composition would permit the maintenance of the well balanced intestinal bacterial flora. As a result, the offensive smells of the animal's excretion can significantly be reduced, this in turn permits the reduction of any deterioration of the environment around the facilities for breeding animals due to the generation of such bad smells. Moreover, the foregoing would permit the foundation of such facilities for breeding animals in areas in the proximity to big cities and this may likewise result in the achievement of such an effect that the cost required for the shipping of livestock products can considerably be reduced.

### Best Mode for Carrying Out the Invention

The present invention will hereunder be described in more detail.
The term "silver-carrying zeolite" as used in the present invention means zeolite carrying silver having antimicrobial activity in its stable form. In this connection, examples of such zeolite usable herein include naturally occurring products such as zeolite A, zeolite X, zeolite Y, zeolite T, zeolite having a high silica content, sodalite, mordenite, analcime, clinoptilolite, chabazite and erionite; semi-synthesized products called artificial zeolite; and chemically synthesized products.
Examples of antimicrobial agents usable herein as carriers for silver also include silver-containing glass, zirconium silver phosphate, silver-containing silica gel, silver- containing apatite, fine particles of silver, protein-silver, sulfur diazine-silver, in addition to zeolite type ones such as those listed above, but the use of zeolite type ones is preferred in the present invention because they can ensure the achievement of a stable antimicrobial activity.

Then, a method for preparing such a silver-carrying zeolite product will be described below in detail.
The silver-carrying zeolite used in the present invention can be prepared according to a wet or dry technique. The wet technique comprises, for instance, following steps: zeolite is introduced into a silver ion-containing solution prepared in advance to thus allow the occurrence of ion exchange between silver ions and those present in the zeolite and exchangeable with the former in the liquid phase. This ion-exchange may be carried out according to the batch-wise or continuous method (for instance, a column method) and the operation may be conducted at a temperature ranging from 10 to 70°C and preferably 40 to 60°C, for 3 to 32 hours and preferably 10 to 24 hours. In this respect, it is suitable to adjust the pH value of the silver ion-containing solution to a level ranging from 3 to 10 and preferably 5 to 7. The silver ion-containing solution used herein is in general prepared from any silver salt such as silver nitrate, silver sulfate, silver perchlorate, silver acetate, diammine silver nitrate and diammine silver sulfate.

The amount of the silver ions to be applied to or deposited on zeolite can be adjusted by controlling the silver ion concentration present in the silver ion-containing solution used in the foregoing ion-exchange reaction. When using a silver ion-containing solution having a silver ion concentration falling within the range of from 0.005 to 0.15 M/L, various silver-carrying zeolite products can appropriately be prepared, which have a desired silver ion content ranging from 0.1 to 5.0% by mass on the basis of the total mass of the silver-carrying zeolite. In this connection, the silver ion-containing solution used in the ion-exchange reaction with zeolite may likewise comprise cations other than silver ions such as zinc ions, copper ions, manganese ions, nickel ions, magnesium ions, calcium ions and/or ammonium ions in order to improve the chemical stability of the resulting silver-carrying zeolite. Various kinds of these additional cations can easily be applied to zeolite by the addition of these cations to the silver ion-containing solution. In this respect, it is in general suitable that the various kinds of cations other than silver ions to be applied onto or deposited on the zeolite is controlled to the range of from 0 to about 4 moles per unit mole of silver ions.

On the other hand, the dry technique usable herein may comprise, for instance, the steps of mixing zeolite particles with silver salt particles and then heat-treating the resulting mixture to remove the anions present in the silver salt and to thus give silver-carrying zeolite particles. Alternatively, a silver salt solution prepared by dissolving the silver salt in a small amount of water can be substituted for the silver salt particles used in the foregoing dry technique to thus give silver-carrying zeolite particles. In this connection, the heat-treatment may suitably be carried out at a temperature ranging from 200 to 700°C and preferably 400 to 600°C, for 20 to 120 minutes and preferably about 30 to 50 minutes. Silver salts usable in the dry technique may be, for instance, those listed above in connection with the wet technique. Similarly, zeolite particles carrying cations other than silver ions together with the latter can be prepared by the use of an aqueous solution containing a salt other than a silver salt in addition to the latter, like the foregoing wet technique.

In the present invention, the amount of silver supported by or included in the zeolite preferably ranges from 0.1 to 5.0% by mass and more preferably 0.5 to 2.5% by mass on the basis of the total mass of the silver-carrying zeolite, from the viewpoints of the antibiotic activity against the bacteria or the like present in animal bodies and the safety thereof.
The amount of the silver-carrying zeolite particles to be incorporated into the feed composition of the present invention preferably ranges from 0.001 to 0.2% by mass and more preferably 0.01 to 0.1% by mass on the basis of the total mass of the resulting feed composition, in the light of the antibiotic activity against the bacteria or the like present in animal bodies and the safety of the composition to the animal body.
The content of silver-carrying zeolite to be incorporated into the feed composition of the present invention may fall within the range specified above when the composition is directly fed or administered to animals or it preferably ranges from 0.001 to 0.2% by mass and more preferably 0.01 to 0.08% by mass on the basis of the total mass of the resulting feed composition.
Moreover, the feed composition of the present invention may likewise be prepared in the form of a concentrated feed composition and the latter may be diluted with the usual feed composition such that the resulting mixture has a silver content specified above before administering the same to animals. The silver-carrying zeolite content in the concentrated feed composition preferably ranges from 0.1 to 10% by mass and more preferably 0.5 to 5% by mass on the basis of the total mass of the concentrated feed composition.

The feed composition of the present invention may further comprise optional components such as a various kinds of amino acids, minerals, enzymes and/or lactic acid bacteria, in addition to the foregoing components for the supplementation of nutrients, the nutritional enrichment, the improvement of digestion and absorption, the promotion of growth and the impartment of disease resistance.
The feed composition of the present invention can easily be prepared by admixing powdery raw materials in a mixer. In this respect, the particle size of each raw material is not restricted to any particular level and it can, if necessary, be adjusted while taking into consideration each particular application or the kinds of animals to be bred.

According to another aspect of the present invention, there is also provided a method for breeding animals characterized by administering a feed composition containing silver-carrying zeolite particles.

The animals to be bred according to the method of the present invention are not restricted to specific ones.
The animals usable in the breeding method of the present invention may be, for instance, a variety of animals bred or cultivated as, for instance, livestock, poultry and pet animals and specific examples thereof include mammals, birds, fishes, reptiles and batrachians. The animals to which the feed composition of the present invention can suitable be administered include, for instance, those which can provide, to man, various valuable substances such as meat, eggs, milk, hair, furs, feathers, down, fells and/or skins.
Examples of animals for obtaining meat are domestic animals and domestic fowls such as chickens, cattle, swine, goats, equines, sheep, deer, bears, reindeer, rabbits, ostriches, domestic ducks, turkeys and quails; and cultivated fishes such as trout, young yellowtails and eels.
Examples of animals for egg collection are birds and fishes listed above in connection with the animals for obtaining meat.
Examples of animals for collecting milk usable herein are caws, she-goats and equines.
Examples of animals for collecting hairs (or fur, feathers or downs), furs or skins include cattle, equines, swine, sheep, goats, deer, bears, reindeer, dogs, cats, rabbits, minks, martens, foxes, raccoon dogs and birds for collecting feathers and/or downs.
The animals to which the feed composition of the present invention can suitably be administered include, for instance, animals for obtaining meat and those for egg collection among the animals specifically listed above and specific examples thereof are domestic animals and domestic fowls such as chickens for obtaining meat (chicken), beef cattle, cattle for collecting milk and meat, swine, goats, equines, rabbits, ostriches, domestic ducks, turkeys and quails and, in particular, chickens for obtaining meat (chicken), chickens for egg collection, beef cattle, swine and ostriches.
Further, when the feed composition according to the present invention is administered to pet animals such as small birds and small animals; aquarium fishes such as carps, goldfishes and tropical fishes; laboratory animals such as rats and mice; and various kinds of animals kept in zoological gardens, these animals can be protected beforehand from the possible infection with any infectious pathogenic bacteria and/or viruses.

The animal-breeding method of the present invention is characterized in that a feed composition is administered to each animal in such a manner that the content of the silver-carrying zeolite is changed depending on the different growth periods of the animal and more specifically, the method is characterized in that a feed composition having a relatively high content of silver-carrying zeolite is administered to an animal during the term extending from its birth to its childhood until the animal acquires the immunity almost comparable to that of the imago thereof (hereunder referred to as "initial breeding stage" or "term A") during which the animal has only a low resistance to exogenous pathogenic bacteria and/or viruses (hereunder also simply referred to as "resistance to pathogenic bacteria"). This would accordingly permit the considerable reduction in the attack rate of these animals having only low resistance to pathogenic bacteria and the healthy growth of these animals up to their imagines within a very short period of time. More specifically, the method of the invention is characterized in that a feed composition having a silver-carrying zeolite content of A% by mass (A being not less than 0.000001) is administered to an animal in its initial breeding stage. For instance, when using silver-carrying zeolite particles whose silver content is 2.5% by mass, a feed composition having a silver-carrying zeolite content of 0.001% by mass is administered to an animal in its initial breeding stage. The silver-carrying zeolite content in a feed composition may vary depending on, for instance, the silver content of each specific silver-carrying zeolite used and the kinds of animals to be bred with the composition and the applications of these animals thus bred, but when using silver-carrying zeolite particles having a silver content of 2.5% by mass, the silver-carrying zeolite content in the feed composition is usually not less than 0.001% by mass (or not less than 10 ppm), preferably not less than 0.005% by mass (or not less than 50 ppm), more preferably not less than 0.01% by mass (or not less than 100 ppm) and most preferably not less than 0.02% by mass (or not less than 200 ppm). The upper limit of the silver-carrying zeolite content in the feed composition of the invention is not restricted to any particular value. However, the use or administration of the silver-carrying zeolite particles in an unreasonably high content would not be desirable from the economical standpoint and it may adversely affect the animals to which the feed composition is administered. Accordingly, the content of the silver-carrying zeolite particles in the feed composition of the invention is suitably not more than 0.1% by mass (or 1000 ppm) based on the total mass of the same. In case where the silver content of the silver-carrying zeolite is beyond the level of 2.5% by mass, it is sufficient to appropriately control the silver content in the feed composition in such a manner that it falls within the range specified above.

The foregoing "initial breeding stage" or "term A" may vary depending on the kinds of animals to be bred with the feed composition and the applications of these animals thus bred. For instance, in case of domestic fowls for egg collection, it corresponds to 16 to 20 weeks after the hatching and preferably about 3 to 5 weeks after the hatching; in case of domestic fowls for obtaining chicken (broiler), it corresponds to 18 to 25 days after the hatching and preferably about 14 to 21 days after the hatching; in case of swine, it corresponds to the term elapsed from its birth to the instance at which the body weight thereof reaches at least 30% by mass and preferably at least about 25% by mass on the basis of the body weight of the imago thereof, for instance, if the body weight of the imago thereof is 120 kg, the term required till the body weight reaches at least 36 kg and preferably at least about 30 kg; in case of beef cattle, it corresponds to the term elapsed from its birth to the instance at which the body weight thereof reaches at least 25% by mass and preferably at least about 38% by mass on the basis of the body weight of the imago thereof, for instance, if the body weight of the imago of beef cattle is 800 kg, it corresponds to the term required till the body weight reaches at least about 200 kg, preferably at least about 300 kg and more preferably at least about 400 kg.

In a preferred embodiment of the breeding method according to the present invention, a composition having a silver content of A% by mass (A being not less than 0.000001) is administered to an animal in its initial breeding stage and a composition having a silver content of B% by mass (B<A) is administered to the animal after the completion of the initial breeding stage. For instance, when using silver-carrying zeolite particles having a silver content of 2.5% by mass, a composition having a silver-carrying zeolite content of not less than 0.001% by mass is administered to an animal in its initial breeding stage and a composition having a silver-carrying zeolite content of less than 0.001% by mass is administered to the animal during a desired term after the completion of the initial breeding stage (hereunder also referred to as "term B").
The silver content in the feed composition administered to an animal during the term B may vary depending on the kinds of animals to be bred with the composition and the applications of these animals thus bred, but it would be sufficient that the silver content satisfies the following requirement: [the silver content in the composition administered during the term A] > [the silver content in the composition administered during the term B]. In this respect, when using silver-carrying zeolite particles having a silver content of 2.5% by mass, the feed composition is usually administered in an amount of less than 0.02% by mass (less than 200 ppm) as expressed in terms of the amount of the silver-carrying zeolite, while the lower limit is not particularly specified and B may be equal to zero. In general, however, the dose of the silver preferably ranges from 0.0001 to 0.05% by mass (1 to 500 ppm) and more preferably 0.001 to 0.01% by mass (10 to 100 ppm) as expressed in terms of the amount of the silver-carrying zeolite incorporated into the feed composition. In case where the silver content of the silver-carrying zeolite is beyond the level of 2.5% by mass, it is sufficient to appropriately control the silver content in the feed composition in such a manner that it falls within the range specified above.

The foregoing "term B", which corresponds to the desired term after the completion of the initial breeding stage, may vary depending on the kinds of animals to be bred with the feed composition and the applications of these animals thus bred. For instance, in case of domestic fowls for egg collection, it corresponds to the term elapsed from the end of the term A or the end of the term: 16 to 20 weeks after the hatching and preferably about 3 to 5 weeks after the hatching, to the selection of chickens (in general about 2 years); in case of domestic fowls for obtaining chicken (broiler), it corresponds to the term elapsed from the end of the term: 18 to 25 days after the hatching and preferably about 14 to 21 days after the hatching to the shipping or forwarding date of the same, preferably 5 to 20 days before the shipping date and more preferably 6 to 8 days before the shipping date; in case of beef cattle and swine, it corresponds to the term elapsed from the end of the term A to the shipping or forwarding date thereof, preferably 5 to 20 days before the shipping date and more preferably 6 to 14 days before the shipping date.

In a further preferred embodiment of the breeding method of the present invention, a feed composition having a silver content of A% by mass (A being not less than 0.000001) is administered to an animal in the initial breeding stage of the animal (during the term A); a feed composition having a silver content of B% by mass (B<A) is administered to the animal during the term elapsed from the end of the initial breeding stage to the shipping date and preferably 5 to 20 days before the shipping date; and a feed composition having a silver content of C% by mass (C>B) is administered to the animal during the term of 5 to 20 days before the shipping date. For instance, when using silver-carrying zeolite particles having a silver content of 2.5% by mass, the feed composition having a silver-carrying zeolite content of not less than 0.001% by mass is administered to the animal in the initial breeding stage thereof; the feed composition having a silver-carrying zeolite content of less than 0.001% by mass is administered to the animal during the term elapsed from the end of the initial breeding stage to 5 to 20 days before the shipping date (or during the term B); and the feed composition having a silver-carrying zeolite content of not less than 0.001% by mass is administered to the animal during the term of 5 to 20 days before the shipping date (hereunder also referred to as "term C").
The silver content in the feed composition administered to an animal during the term C may vary depending on the kinds of animals to be bred with the composition and the applications of these animals thus bred, but it would be sufficient that the silver content satisfies the following relation: [the silver content in the composition administered during the term C] > [the silver content in the composition administered during the term B]. In this respect, when using silver-carrying zeolite particles having a silver content of 2.5% by mass, the feed composition is usually administered in an amount of not less than 0.001% by mass (not less than 10 ppm), preferably not less than 0.005% by mass (not less than 50 ppm), more preferably not less than 0.01% by mass (not less than 100 ppm) and most preferably not less than 0.02% by mass (not less than 200 ppm) as expressed in terms of the amount of the silver-carrying zeolite, while the upper limit of the silver content in the feed composition administered during the term C is not particularly specified. However, the use or administration of the silver-carrying zeolite particles in an unreasonably high content would be less economical and it may adversely affect the animals to which the composition is administered. Accordingly, when using silver-carrying zeolite particles having a silver content of 2.5% by mass, the content of silver should be not more than 0.1% by mass (or not more than 1000 ppm) as expressed in terms of the amount of the silver-carrying zeolite incorporated into the composition. In case where the silver content of the silver-carrying zeolite is beyond the level of 2.5% by mass, it is sufficient to appropriately control the silver content in the feed composition in such a manner that it falls within the range specified above.
The silver content A may be equal to the silver content C and in this case, the feed composition having the same silver-carrying zeolite content can be used during the both terms A and C.

For instance, the term A (the initial breeding stage) corresponds to the term extending from 0 to 21 days after its birth; the term B (the intermediate breeding stage) extends from 22 to 35 days after the birth; and the term C (the term immediately before the shipping) extends from 36^{th} day to the shipping date for the domestic fowls for obtaining meat or chicken.
Moreover, in case of beef cattle, the term A (the initial breeding stage) corresponds to the term during which the body weight of the animal is not more than 400 kg and in general not more than about 300 kg; the term B (the intermediate breeding stage) corresponds to the term during which the body weight of the animal ranges from 300 to 600 kg; and the term C (the term immediately before the shipping) corresponds to the term during which the body weight of the animal is not less than 500 kg and in general about 600 to 700 kg.
On the other hand, in case of swine, the term A (the initial breeding stage) corresponds to the term during which the body weight of the animal is not more than 30 kg; the term B (the intermediate breeding stage) corresponds to the term during which the body weight of the animal ranges from 20 to 70 kg and usually about 30 to 60 kg; and the term C (the term immediately before the shipping) corresponds to the term during which the body weight of the animal ranges from 60 to 120 kg.
The animal-breeding method of the present invention is characterized by dividing the growth period of each animal into the following three terms: the term A (the initial breeding stage), the term B (the intermediate breeding stage) and the term C (the term immediately before the shipping) and administering, to the animal, feed compositions having different silver-carrying zeolite contents depending on the different growth or breeding terms. In other words, a feed composition having a high silver-carrying zeolite is administered to an animal in the term during which the animal has a relatively low resistance to pathogenic bacteria to thus prevent the infection of the animal with pathogenic bacteria such as those belonging to the genus Salmonella; a feed composition having a lowest possible amount of the silver content is administered to the animal during the term wherein the animal acquires sufficiently high resistance to pathogenic bacteria as a result of sufficient growth to thus prevent the infection of the animal with any pathogenic bacteria economically efficiently and to promote and/or maintain the healthy growth and activity of the animal.

### Examples

The present invention will hereunder be described in more detail with reference to the following non-limitative working Examples, but the present invention is not restricted to these specific Examples at all.

### Preparation Example 1: Preparation of Silver-Carrying Zeolite and Feed Composition Containing the Same

Silver-carrying zeolite particles were prepared as follows: There was added water to 1 kg of powder obtained by drying commercially available Zeolite A Na₂O·Al₂O₃·2SiO₂·xH₂O; average particle size: 1.5 µm) at 110°C to thus give 1.3 L of a slurry and then the slurry was stirred at 20°C for 18 hours. Appropriate amounts of a nitric acid aqueous solution and water were added to the slurry to control the pH value thereof to a level ranging from 5 to 7. Then the slurry was poured into 3 L of a 0.075 M/L aqueous solution of silver nitrate and the resulting mixture was continuously stirred at 50°C for 24 hours to thus establish an equilibrium condition. After the completion of the reaction, the zeolite phase was filtered off and washed with water to remove, for instance, excess silver ions. The resulting zeolite phase was dried at 110°C to thus give silver-carrying zeolite particles. The silver content of the resulting zeolite particles was found to be 2.5% by mass.
Thereafter there were prepared various kinds of feed compositions containing the resulting silver-carrying zeolite particles incorporated therein. Examples of the formulations thereof are summarized in the following Table 1. In this respect, there were also prepared comparative samples which were free of any silver-carrying zeolite particles or to which ampicillin as a typical antibiotic agent was incorporated.

### Test Examples 1 to 5: Tests for Confirming the Effect of Feed Composition Prepared Above by Administering It to Domestic Fowls for Obtaining Chicken

Tests were carried out using domestic fowls for obtaining chicken for the confirmation of the effects of the feed compositions prepared in Preparation Example 1 and listed in Table 1. In the following, the term "part" means "part by mass" unless otherwise specified.
These tests were carried out using 5 test groups (including 1,400 domestic fowls in all; each corresponds to Tests 1 to 5) each comprising 7 test subgroups (comprising 280 fowls) wherein each subgroup consisted of 40 domestic fowls including hens and cocks (20 domestic fowls each). The experimental result for each test group was defined to be the average of the corresponding 7 subgroups wherein the average value for each subgroup corresponded to the average value of the fowls constituting each subgroup. In these tests, the term A (the initial breeding stage) was defined to be the term elapsed from 0 to 21 days after the birth of these fowls; the term B (the intermediate breeding stage) was defined to be the term elapsed from 22 to 35 days after the birth thereof; and the term C (the term immediately before the shipping) was defined to be the term elapsed from 36 to 42 days after the birth thereof. and these test were completed after 42 days from the birth thereof. The following Table 2 shows the details of the feed compositions administered to every test groups during every corresponding growth periods.

The domestic fowls in each test group were evaluated by inspecting them for the following various characteristic properties: the rate (%) of body weight increase, the feed conversion rate, the score of lesions, and the number of bacterial cells of Salmonella found in the feces of each fowl. The results thus obtained are summarized in the following Table 3.
Body Weight Increase (g): This is herein defined to be the difference obtained by subtracting the body weight observed when the initiation of the test from that observed at the end of the test.
Feed Conversion Rate: This is defined to be the value obtained by dividing the overall amount of the feed composition fed to each fowl during the test by the body weight increase observed for the same.
Score of Lesions: This is an indication for evaluating the healthiness of the intestinal tracts of the domestic fowls for chicken. More specifically, this is evaluated by observing the surface of the intestinal tract from the exterior thereof through the thin membrane (serous membrane) covering the tract while irradiating the intestinal tract with light rays from a fluorescent lighting. In addition, this was likewise evaluated by dissecting the intestinal tract to thus examine the contents thereof on the effects, on the same, of E. tenella (coccidia) as one of the typical harmful microorganisms. The effects of the bacteria were judged by a veterinarian according to the following criteria:
0: There is not observed any lesion at all;
+1: There is observed quite slight scattering of spot-like bleeding flecks on the wall of the cecum, but there is not observed any thickening of the intestinal wall. The contents thereof are normal.
+2: There is observed slight contamination of the contents with the blood and a large number of hemorrhagic lesions. The wall of the cecum is slightly thickened.
+3: There are observed a large amount of blood or the cores of cecum (coagulated products or ash-colored cheese-like and banana-shaped clotted blood) in the cecum and there are clearly observed the thickening of the cecum wall as well as definite deformation and contraction of the cecum.
+4: The cecum is considerably contracted and the lesion reaches even the rectum area. The wall of the cecum is extremely thickened and the cecum includes clotted blood or cores of cecum. In this respect, if the lesions are not uniform on the both sides of the cecum, the evaluation thereof should be carried out on the basis of the severe one.

Number of Bacterial Cells of Salmonella Present in Feces: This was evaluated according to the following criteria:
High Rate of Risk (++): Not less than 10³ cfu/g; It would be highly possible that the product is contaminated and it in turn becomes a cause of various defects from the viewpoint of the food sanitation.
Some Risk May be Present (+): Not less than 10² cfu/g; There would be such a possibility that the product is contaminated and it in turn becomes a cause of defects from the viewpoint of the food sanitation.
Almost No Risk (-): Less than 10² cfu/g; There is hardly any such a possibility that the product is contaminated and it in turn becomes a cause of defects from the viewpoint of the food sanitation.

**Table 1:**

| Feed | Main Component of Feed | Antibiotic Component | Other Components Added |
|---|---|---|---|
| Z1 | Corn Particles/Soybean: 100 parts | Silver-Carrying Zeolite: 0.01 part | Amino acids, minerals: 4 parts |
| Z2 | Corn Particles/Soybean: 100 parts | Silver-Carrying Zeolite: 0.02 part | Amino acids, minerals: 4 parts |
| Z3 | Corn Particles/Soybean: 100 parts | Silver-Carrying Zeolite: 0.04 part | Amino acids, minerals: 4 parts |
| Z4 | Corn Particles/Soybean: 100 parts | Silver-Carrying Zeolite: 0.08 part | Amino acids, minerals: 4 parts |
| N1 | Corn Particles/Soybean: 100 parts | None | Amino acids, minerals: 4 parts |
| N2 | Corn Particles/Soybean: 100 parts | Antibiotic Agent: 0.01 part | Amino acids, minerals: 4 parts |

**Table 2:**

| Test Ex. | Breeding Period (Term A) | Breeding Period (Term B) | Term Just Before Shipping (Term C) | Total Dose (g) | Remarks |
|---|---|---|---|---|---|
| 1 | Feed Z1 | Feed Z1 | Feed Z1 | 3480 | Present Invention |
| 2 | Feed Z3 | Feed Z3 | Feed Z3 | 3418 | Present Invention |
| 3 | Feed Z2 | Feed N1 | Feed Z4 | 3425 | Present Invention |
| 4 | Feed N1 | Feed N1 | Feed N1 | 3458 | Control |
| 5 | Feed N2 | Feed N2 | Feed N1 | 3482 | Comp. Example |

**Table 3:**

| Test Ex. | Av. body wt. inc. | Feed conversion rate | Score of lesions | No. of cells of Salmonella (eval.) | Remarks |
|---|---|---|---|---|---|
| 1 | 1887 | 1.860 | 0.857 | 1552 (++) | Present Invention |
| 2 | 1903 | 1.813 | 0.214 | 56 (-) | Present Invention |
| 3 | 1898 | 1.819 | 0.250 | 82 (-) | Present Invention |
| 4 | 1864 | 1.870 | 1.554 | 1774 (++) | Control |
| 5 | 1887 | 1.854 | 0.393 | 167 (-) | Comp. Example |

The foregoing results clearly show that the test examples 2 and 3 show "scores of lesion" lower than those observed for the test example 5 (control) and the test example 6 (Comparative Example), that they are negative in the evaluation (eval.) of the effect of bacteria belonging to the genus Salmonella and that they can accordingly provide highly safe chicken. Moreover, the test example 4 shows the intended effect almost identical to that achieved by the test example 3 although the total dose in the former is lower that that used in the latter.

### Test Example 2: Test for Antibiotic Effect of Silver-Carrying Zeolite

The zeolite particles having a silver content of 2.5% by mass prepared in Preparation Example 1 as well as various kinds of commercially available antimicrobial agents were inspected for the antimicrobial effects on bacteria belonging to the genus Salmonella and lactic acid bacteria which were typical of the useful bacteria.
The antimicrobial effects of these samples were evaluated by the determination of the minimum growth-inhibitory concentration (MIC) and the minimal bactericidal concentration (MBC). This method is known as one approved by Chemotherapeutic Society of Japan.
In this connection, bacterial strains used herein were those isolated from the intestinal tracts of domestic fowls for obtaining chicken. The results thus obtained are listed in the following Table 4:

**Table 4:**

| Antimicrobial Agent | Bacterial Cells of Salmonella | | Lactic Acid Bacteria | |
|---|---|---|---|---|
| | MIC | MBC | MIC | MBC |
| Ag-Carrying Zeolite | 125 | 62 | 1000 | 1000 |
| Silver sulfadiazine | 1000 | 500 | 500 | 125 |
| Ampicillin | >2000 | >2000 | 2000 | 1000 |

The foregoing data listed in Table 4 clearly indicate that when comparing the silver-carrying zeolite with other antimicrobial agents tested herein, the former shows a higher bactericidal effect on the bacterial cells of Salmonella, while it shows only a weaker effect on lactic acid bacteria. Incidentally, it would be recognized that ampicillin as an antimicrobial agent in fact shows a quite weak bactericidal effect on the bacteria belonging to the genus Salmonella since the bacteria has already acquired the resistance thereto.

### Industrial applicability

The feed composition of the present invention has almost no influence upon the useful lactic acid bacteria, but exerts an antimicrobial effect on various kinds of harmful microorganisms, in particular, bacteria belonging to the genus Salmonella or the like. Accordingly, the feed composition of the invention would permit the improvement of the healthiness of intestinal tracts of domestic animals, domestic fowls and cultivated fishes such as chickens, swine, cattle, sheep, equines, carps and various kinds of cultivated fishes and the composition would in its turn permit the acceleration of their growth, while maintaining their good healthiness.

## Claims

1. A feed composition **characterized in that** it comprises silver-carrying zeolite.

2. The feed composition of claim 1, wherein the amount of silver present in the silver-carrying zeolite ranges from 0.1 to 5.0% by mass on the basis of the total mass of the silver-carrying zeolite.

3. The feed composition of claim 1, wherein the content of silver in the composition ranges from 0.000001 to 0.2% by mass (0.01 to 2000 ppm) on the basis of the total mass of the feed composition.

4. The feed composition of claim 1, wherein the amount of silver present in the silver-carrying zeolite ranges from 0.1 to 5.0% by mass on the basis of the total mass of the silver-carrying zeolite and the content of silver in the feed composition ranges from 0.000001 to 0.2% by mass (0.01 to 2000 ppm) on the basis of the total mass of the composition.

5. A method for breeding an animal comprising the step of administering, to an animal, a feed composition comprising silver-carrying zeolite.

6. The animal-breeding method of claim 5, wherein the amount of silver present in the silver-carrying zeolite ranges from 0.1 to 5.0% by mass on the basis of the total mass of the silver-carrying zeolite.

7. The animal-breeding method of claim 5, wherein the content of silver in the composition ranges from 0.000001 to 0.2% by mass (0.01 to 2000 ppm) on the basis of the total mass of the feed composition.

8. The animal-breeding method of claim 5, wherein the amount of silver present in the silver-carrying zeolite ranges from 0.1 to 5.0% by mass on the basis of the total mass of the silver-carrying zeolite and the content of silver in the feed composition ranges from 0.000001 to 0.2% by mass (0.01 to 2000 ppm) on the basis of the total mass of the composition.

9. The animal-breeding method as set forth in any one of claims 5 to 8, wherein the feed composition having a silver content of A% by mass (A being not less than 0.000001) is administered to the animal in the initial breeding stage.

10. The animal-breeding method as set forth in any one of claims 5 to 8, wherein the composition having a silver content of A% by mass (A being not less than 0.000001) is administered to the animal in the initial breeding stage and the composition having a silver content of B% by mass (B<A) is administered to the animal after the completion of the initial breeding stage.

11. The animal-breeding method of claim 10, wherein the animal is a fowl bred for egg collection or dairy cattle.

12. The animal-breeding method as set forth in any one of claims 5 to 8, wherein the composition having a silver content of A% by mass (A being not less than 0.000001) is administered to the animal in the initial breeding stage; the composition having a silver content of B% by mass (B<A) is administered to the animal during the term extending from the end of the initial breeding stage to 5 to 20 days before the distribution or shipping date; and the composition having a silver content of C% by mass (C>B) is administered to the animal during the term of 5 to 20 days before the shipping date.

13. The animal-breeding method of claim 12, wherein the animal is one for obtaining meat.

14. The animal-breeding method of claim 13, wherein the animal is poultry, cattle, swine, a wild boar, an equine, sheep or a deer.

15. The animal-breeding method of claim 13, wherein the animal is a domestic fowl for chicken, beef cattle or swine.
